# EUROPEAN PATENT SPECIFICATION

(11) **EP 4 020 382 B1**
(45) Date of publication and mention of the grant of the patent: **22.10.2025**
(21) Application number: 21215772.1
(22) Date of filing: 17.12.2021
(51) Int. Cl.: G06T 7/00

(54) **EVALUATION METHOD**
AUSWERTUNGSVERFAHREN
PROCÉDÉ D'ÉVALUATION

(30) Priority: 23.12.2020 JP 2020213410
(43) Date of publication of application: 29.06.2022
(73) Proprietor: Ricoh Company, Ltd., Tokyo 143-8555 (JP)
(72) Inventor: MATSUMURA, Takashi, Tokyo 143-8555 (JP); NIIMI, Tatsuya, Tokyo 143-8555 (JP); SAITO, Takuya, Tokyo 143-8555 (JP)
(74) Representative: Marks & Clerk LLP

(56) References cited:
- WANG LING ET AL: "Automated quantitative assessment of three-dimensional bioprinted hydrogel scaffolds using optical coherence tomography.", BIOMEDICAL OPTICS EXPRESS 01 MAR 2016, vol. 7, no. 3, March 2016 (2016-03-01), pages 894 - 910, XP055916144, ISSN: 2156-7085
- RAMESH SRIKANTHAN ET AL: "Extrusion bioprinting: Recent progress, challenges, and future opportunities", BIOPRINTING, vol. 21, 23 November 2020 (2020-11-23), pages e00116, XP055916149, ISSN: 2405-8866, DOI: 10.1016/j.bprint.2020.e00116
- DIMITRIS MITSOURAS ET AL: "Three-dimensional printing of MRI-visible phantoms and MR image-guided therapy simulation", MAGNETIC RESONANCE IN MEDICINE, 11 February 2016 (2016-02-11), US, pages n/a - n/a, XP055303754, ISSN: 0740-3194, DOI: 10.1002/mrm.26136

## Description

### BACKGROUND

### Technical Field

The present disclosure relates to an evaluation method.

### Description of the Related Art

Three-dimensional object producing techniques have made a remarkable progress and various three-dimensional ("3D") printers of a fused deposition modeling type, a binder jetting type, a stereolithography type, a powder sintering additive manufacturing type, and a material jetting type have been used in the manufacturing industry. As the materials used in the 3D printers, various materials have been being developed, including metals and resins that have been hitherto known. New uses suited to these various materials have also been proposed, and applications of 3D printers to, for example, the medical field and the healthcare field have been being expected, as well as to the industrial field. Examples of applications of 3D printers to the medical field include production of implantable artificial bones using, for example, titanium, hydroxyapatite, and PEEK, and studies into artificial organs obtained by direct lamination of cells in layers. Examples of applications of 3D printers to the healthcare field include applications to, for example, hearing aids and artificial limbs that need to reflect individual-specific shapes. What is behind this expanded ranges of applications is easy availability of 3D data by use of, for example, 3D scanners, CT, and MRI.

Other examples of expected applications of 3D printers to the medical field include applications to three-dimensional objects that imitate actual organ shapes and organisms for surgical trainings and simulations. Within the background context of the expectation for applications to these three-dimensional objects, there are the recent progress in the development of medical devices and the accompanying ongoing shift in the medical trend from the existing medicine including major incisions and excisions to low-invasive, low patient-burdening medicine by, for example, catheters, endoscopes, and robotic assists, and the need for very high-level techniques and skills in the medical operations including use of the mentioned medical devices and the accompanying recognition of the importance of surgical trainings using suitable three-dimensional objects for preventing medical accidents. Moreover, for difficult surgeries with few actual cases done, if three-dimensional objects that reproduce the details of the target sites can be obtained beforehand, it is possible to perform scrupulous preoperative simulations.

Examples of known three-dimensional objects that imitate, for example, organs include three-dimensional objects produced by 3D printers and formed of hard materials such as acrylic-based resins and urethane-based resins, and three-dimensional objects produced by a casting method using a template and formed of water-based gels such as silicone elastomers and polyvinyl alcohols disclosed in JP-2015-069054-A. Other examples include three-dimensional objects produced by 3D printers and formed of hydrogels disclosed in JP-2017-26680-A. However, even when produced based on 3D data of the target organs, these three-dimensional objects cannot reproduce the organs perfectly.

Moreover, the target organs are in the patients' bodies, and it is difficult to measure the shapes of the organs. Therefore, even if three-dimensional objects imitating the organs are produced, it is difficult to evaluate how accurately the three-dimensional objects reproduce the shapes of the actual organs.

WO 2018/143444 A1 discloses a method for comparing the shape of an internal organ represented by organ 3D data obtained by measuring the internal organ with a CT scanner with a model shape represented by model 3D data obtained by measuring a model with a CT scanner.

However, a CT scanner has a relatively low density resolution and cannot minutely measure shapes of, for example, blood vessels inside an organ. Therefore, it is difficult to minutely evaluate the accuracy of a three-dimensional object with respect to an actual organ.

Ling Wang et al., "Automated quantitative assessment of three-dimensional bioprinted hydrogel scaffolds using optical coherence tomography," Biomed. Opt. Express 7, 894-910 (2016), discloses a method of reconstructing and quantitatively assessing the internal architecture of opaque three-dimensional bioprinted hydrogel scaffolds using swept-source optical coherence tomography. Ramesh, S et al., "Extrusion bioprinting: Recent progress, challenges, and future opportunities", Bioprinting 21, [e00116] (2021), discloses a review highlighting the role of rheology and process parameters in extrusion bioprinting and discusses qualitative and quantitative methods proposed to measure and define the printability of bioinks.

### SUMMARY

The present disclosure has an object to provide an evaluation method for minutely evaluating accuracy of a three-dimensional object imitating an organism with respect to the organism.

An aspect of the present disclosure provides an evaluation method according to claim 1.

The present disclosure can provide an evaluation method for evaluating accuracy of a three-dimensional object imitating an organism with respect to the organism.

### BRIEF DESCRIPTION OF THE SEVERAL VIEWS OF THE DRAWINGS

A more complete appreciation of the disclosure and many of the attendant advantages and features thereof can be readily obtained and understood from the following detailed description with reference to the accompanying drawings, wherein:
FIG. 1 is an exemplary view illustrating an example of a medical image data set;
FIG. 2 is an exemplary view illustrating an example of medical 3D data;
FIG. 3 is an exemplary view illustrating an example of medical 3D data;
FIG. 4 is an exemplary diagram illustrating an example of region-specific medical 3D data obtained by dividing medical 3D data on a voxel region basis;
FIG. 5 is an exemplary diagram illustrating an example of STL-format data obtained by converting region-specific medical 3D data to a surface model;
FIG. 6 is an exemplary diagram illustrating an example of FAV-format data obtained by converting region-specific medical 3D data to a FAV format;
FIG. 7 is an exemplary diagram illustrating an example of a seven-gradation strength property expressed by arrangement patterns of a high-strength object producing composition and a low-strength object producing composition;
FIG. 8 is an exemplary view illustrating an example of a water-swellable layered clay mineral serving as a mineral, and a dispersed state of the water-swellable layered clay mineral in water;
FIG. 9 is an exemplary view illustrating an example of a three-dimensional hydrogel object producing apparatus; and
FIG. 10 is an exemplary view illustrating an example of a three-dimensional hydrogel object detached from supports.

The accompanying drawings are intended to depict embodiments of the present invention and should not be interpreted to limit the scope thereof. The accompanying drawings are not to be considered as drawn to scale unless explicitly noted.

### DETAILED DESCRIPTION

The terminology used herein is for the purpose of describing particular embodiments only and is not intended to be limiting of the present invention. As used herein, the singular forms "a", "an" and "the" are intended to include the plural forms as well, unless the context clearly indicates otherwise.

In describing embodiments illustrated in the drawings, specific terminology is employed for the sake of clarity. However, the disclosure of this specification is not intended to be limited to the specific terminology so selected and it is to be understood that each specific element includes all technical equivalents that have a similar function, operate in a similar manner, and achieve a similar result.

### 1. Evaluation method

An evaluation method of the present disclosure is an evaluation method for evaluating accuracy of a three-dimensional object imitating an organism (hereinafter, may also be referred to as a "model") with respect to the organism. The evaluation method includes an evaluation step of evaluating the accuracy of the model with respect to the organism based on organism shape information representing an organism shape and model shape information representing a model shape. As needed, the evaluation method of the present disclosure may include, as steps performed before the evaluation step, an organism shape information obtaining step of obtaining organism shape information and model shape information obtaining step of obtaining model shape information.

In the present disclosure, an "organism" represents at least a part that physically constitutes a human or a living thing other than a human. An organism may be a part integrated as one entity, or may be a plurality of separate parts. A model that is substantially the same as an organism is also encompassed within "organism". At least a part that constitutes a human or a living thing other than a human represents, for example, a predetermined region within an organism, and a predetermined organ, a predetermined blood vessel, and a predetermined tissue such as a muscle tissue within an organism. At least a part that constitutes a human or a living thing other than a human may be one that has been excised from a human or a living thing other than a human. The predetermined region within an organism encompasses a region that is medically recognized as one region such as chest, abdomen, head, trunk, upper body, and lower body, or combinations thereof, and one region obtained by imaginarily dividing an organism in an arbitrary direction (for example, an axial or transverse direction, a sagittal direction, and a coronal direction). A predetermined organ within an organism is, for example, an organ such as heart and liver, but may be a part of an organ such as a blood vessel and a valve.

In the present disclosure, a "model" means a three-dimensional object imitating an organism. Such a model may imitate a specific target organism or may imitate a typical shape, or may be generated based on shape data of, for example, a specific target organism or may be generated based on average data of shapes of living bodies of a plurality of humans.

### (1) Organism shape information obtaining step

It is preferable that the evaluation method of the present disclosure include an organism shape information obtaining step of obtaining organism shape information as a step performed before the evaluation step. It is preferable that the organism shape information be obtained as information included in medical 3D data. In the present disclosure, "medical 3D data" means data generated based on medical image data obtained by capturing an image of an organism with a medical image capturing device.

In the present disclosure, "organism shape information" is information representing a shape of an organism, obtained by measuring the organism with a medical image capturing device (hereinafter, measuring may be referred to as "capturing an image"). A shape of an organism is a concept including an external shape of the organism and an internal shape of the organism. An "external shape of an organism" represents the shape of the external surface of the organism. When an organism is an organ, an "external shape of the organism" represents the shape of the external surface of the organ or a bone. When an organism is a predetermined region within an organism, an "external shape of the organism" represents the shape of the external surface of each organ or bone included within the region. An "internal shape of an organism" represents the shape of an internal element within the organism, and represents the shape of, for example, a blood vessel. Further, when an organism is, for example, an organ, an internal shape of the organism represents the shape of a blood vessel within the organ. For example, when an organism is a liver, an internal shape of the organism represents the shape of a blood vessel or a gall bladder within the liver. When an organism is a predetermined region within an organism, an "internal shape of the organism" represents the shape of a blood vessel within each organ included within the region. For example, when an organism is a chest, an internal shape of the organism represents the shape of, for example, a blood vessel within, for example, a heart or a lung.

Hence, as an embodiment of the organism shape information obtaining step, a medical 3D data obtaining step of obtaining medical 3D data will be described. The medical 3D data obtaining step includes a medical image capturing step of capturing an image of an organism with a medical image capturing device to obtain medical image data, and a medical 3D data generating step of generating medical 3D data based on the medical image data and the shape of the organism. The medical 3D data obtaining step may include a biological property measuring step of measuring the organism by MRE to obtain a biological property.

### (i) Medical image capturing step

The medical image capturing step is a step of capturing an image of the organism with a medical image capturing device to obtain medical image data. Through the medical image capturing step, the medical image capturing device obtains organism shape information. The medical image capturing device is also referred to as modality, and is configured to scan an organism serving as a subject and obtain medical image data. Examples of the medical image capturing device include a Computed Tomography (CT) device, a Magnetic Resonance Imaging (MRI) device, and ultrasonic diagnostic equipment.

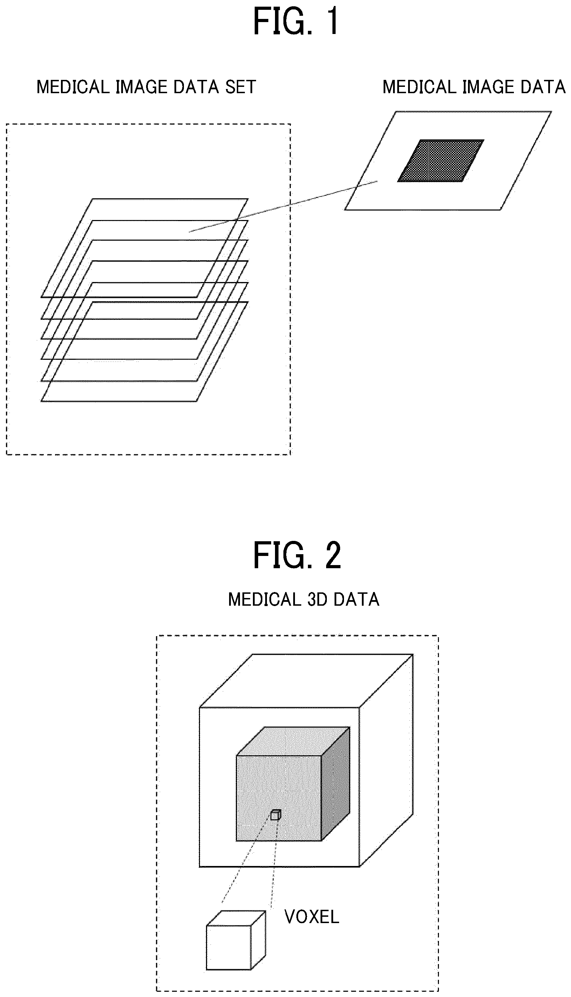

The medical image capturing device performs slice tomography of capturing cross-sectional images of an organism a plurality of times. In this way, the medical image capturing device can obtain a plurality of medical image data, which represent medical images, which are images representing cross-sections of an organism, as illustrated in FIG. 1. It is preferable that each of the plurality of medical image data (also referred to as "medical image data set") be an image of a DICOM-format, which is an international standard relating to medical image exchange. The medical image data include image density information indicating image densities obtained by the medical image capturing device. When a CT device is used as the medical image capturing device, the image density is a CT value (X ray transmittance). When an MRI device is used as the medical image capturing device, the image density is an MRI signal value. When ultrasonic diagnostic equipment is used as the medical image capturing device, the image density is a reflection intensity.

As the medical image capturing device, any of a CT device, an MRI device, and ultrasonic diagnostic equipment may be used for capturing an image of an external shape of an organism. It is suitable to use an MRI device among the medical image capturing devices, because an image of an internal shape of the organism can also be captured in addition to an image of the external shape of the organism because a signal source of an MRI device is a hydrogen nucleus and the organism contains water. It is suitable to use an MRI device also because the MRI device can perform MRE measurement in the biological property measuring step described below. It is suitable to use an MRI device also because the MRI device can capture images without exposing the patient to radiation.

### (ii) Biological property measuring step

The biological property measuring step is a step of obtaining a biological property of an organism such as viscoelasticity or distribution of viscoelasticity by, for example, MRE measurement. MRE measurement represents measurement according to Magnetic Resonance Elastography (MRE) method. This method is a non-invasive method that measures a viscoelasticity distribution in a subject by capturing images of the subject with an MRI device while generating a shear wave inside the subject with an exciter. That is, it is preferable that the biological property obtained by MRE measurement of an organism be viscoelasticity. Examples of the method for obtaining the viscoelasticity of an organism include not only the MRE measurement method, but also an ultrasonic elastography measurement method.

In this regard, information obtained by ultrasonic elastography measurement is one-dimensional information, whereas information obtained by MRE measurement is two-dimensional information. Therefore, MRE measurement is preferable because it is easier to generate 3D data. When the measuring target is a periodically moving organ such as a blood vessel, examples of the method for obtaining a biological property includes a method of measuring the amount of deformation of an organ due to this periodical movement and estimating the biological property from the measured value based on, for example, a dynamical model. However, the biological property obtained by this method is merely an estimated value. Therefore, this method is inferior to MRE measurement in terms of obtaining accurate information of an organism. In addition, this method can be used only when the measuring target is a periodically moving organ such as a blood vessel. Therefore, MRE measurement is preferable because MRE measurement can measure as targets, an organ without periodical movement and an organ of which amount of deformation due to periodical movement is difficult to measure.

MRE measurement is often included in the system of an MRI device as an optional function. Hence, MRE measurement is preferable also because the medical image capturing step and the biological property measuring step can be performed by the same MRI device. Incidentally, the medical image capturing step by an MRI device and the biological property measuring step by an MRI device can be performed almost at the same time. It is preferable to perform these steps almost at the same time, because this makes it possible to reduce burden on the subject such as a patient, and to obtain accurate information of an organism since medical image data and a biological property are obtained almost at the same time. Particularly, the latter is an important aspect, considering that the image-capturing and measuring target is an organism having a nature that, for example, the shape and properties thereof tend to change over time. "Almost at the same time" includes, for example, a case where the period of time for the medical image capturing step and the period of time for the biological property measuring step at least partially overlap, and a case where both of the medical image capturing step and the biological property measuring step can be performed by use of an MRI device once.

The medical image capturing device includes a bed on which the image capturing target person lies. When capturing an image of or measuring an organism such as an excised organ in the medical image capturing step or the biological property measuring step, the organism may be put directly on the bed. However, it is preferable to put the organism indirectly. Indirectly putting the organism means providing a support table between the organism and the bed or putting the organism on the bed in a state that the organism is being dipped in a liquid or a gel. This makes it possible to prevent deformation of the organism due to contact with the bed and to capture an image of or measure the organism in its shape close to when the organism was in the body. The support table needs at least to be able to prevent deformation of the organism due to contact with the bed. Examples of the support table include a soft gel-state table.

### (iii) Medical 3D data generating step

The medical 3D data generating step is a step of generating medical 3D data based on medical image data obtained in the medical image capturing step and biological property information obtained in the biological property measuring step. **In** the present disclosure, "biological property information" is information indicating the biological property obtained by, for example, MRE measurement of an organism.

The medical 3D data is information that includes a plurality of voxels generated based on the medical image data, voxel-by-voxel image density information indicating an image density in the medical image data and allocated to each voxel, and voxel-by-voxel biological property information indicating a biological property allocated to each voxel. In other words, the medical 3D data is information that associates voxels, image density information, and biological property information with one another. Note that the biological property measuring step is an optional step of the medical 3D data generating step. When the biological property measuring step is not provided, medical 3D data may be generated based only on a plurality of voxels generated based on the medical image data, and image density information indicating an image density in the medical image data and allocated to each voxel. In this case, the medical 3D data is information including a plurality of voxels generated based on the medical image data and image density information indicating an image density in the medical image data and allocated to each voxel.

The method for generating the medical 3D data will be specifically described.

First, the medical image capturing device or an image processing device relating to the medical image capturing device (both also referred to as "medical image capturing device, etc.") generates medical 3D data for 3D image processing, from the medical image data obtained in the medical image capturing step. The medical 3D data is an aggregate of voxels including at least one minimum unit such as a cube as illustrated in FIG. 2, and various kinds of information can be associated with each voxel.

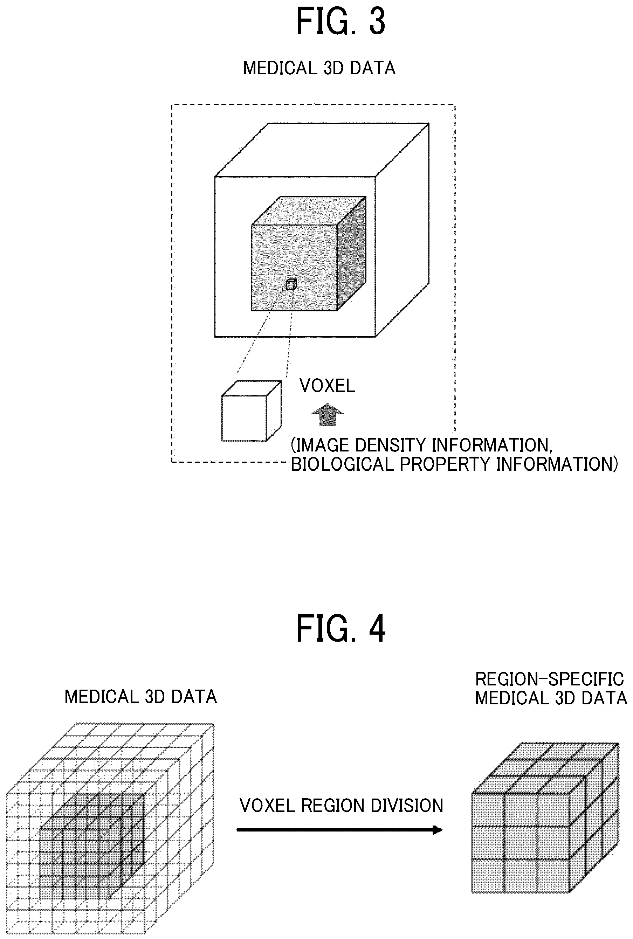

Next, the medical image capturing device, etc. associates image density information indicating an image density in the medical image data obtained in the medical image capturing step with each voxel. Then, the medical image capturing device, etc. associates biological property information indicating the biological property obtained in the biological property measuring step with each voxel. In this way, medical 3D data, which is information associating voxels, image density information, and biological property information with one another, can be generated as illustrated in FIG. 3. Using the image density information, the medical 3D data can be regionally divided (segmented) in the voxel units. Voxel region division means sorting and segmentation (compartmentalization) of voxels having close image densities. This enables, for example, recognition, structural division, tissue analyses, and 3D image analyses of shapes of an organism including an external shape of the organism and an internal shape of the organism based on the medical 3D data, and, for example, makes it possible to obtain medical 3D data of a predetermined tissue such as liver from the medical 3D data of a predetermined region of the organism.

In the present disclosure, as illustrated in FIG. 4, medical 3D data of a partial region obtained by voxel region division of the medical 3D data is referred to as region-specific medical 3D data. Region-specific medical 3D data is one concept encompassed in the medical 3D data. "Organism shape information" is information representing a shape of an organism obtained by measuring the organism by, for example, MRI. Examples of the organism shape information include information associating voxels and image density information with each other in the medical 3D data, and information associating voxels and an organism shape with each other. "Organism shape information" includes "organism external shape information" and "organism internal shape information".

### (2) Model shape information obtaining step

It is preferable that the evaluation method of the present disclosure include a model shape information obtaining step of obtaining model shape information, as a step performed before the evaluation step. It is preferable that model shape information be obtained as information included in model 3D data. In the present disclosure, "model 3D data" represents data generated based on model image data obtained by capturing an image of a model with a medical image capturing device.

In the present disclosure, "model shape information" is information representing a shape of a model, obtained by measuring a model with a medical image capturing device (hereinafter, measuring may be referred to as "capturing an image"). A shape of a model is a concept including an external shape of the model and an internal shape of the model.

An "external shape of a model" represents the shape of the external surface of the model. When a model is an organ or a bone, an "external shape of the model" represents the shape of the external surface of the organ or the bone. When a model is a predetermined region within an organism, an "external shape of the model" represents the shape of the external surface of each organ or bone included within the region. An "internal shape of a model" represents the shape of an internal element within the model, and represents the shape of, for example, a blood vessel. Further, when a model is a model of an organ, an "internal shape of the model" represents the shape of a blood vessel within the organ. For example, when a model is a model of a liver, an internal shape of the model represents the shape of a blood vessel or a gall bladder within the liver. When a model is a model of a predetermined region within an organism, an "internal shape of the model" represents the shape of a blood vessel within each organ included within the region. For example, when a model is a model of a chest, an internal shape of the model represents the shape of, for example, a blood vessel within, for example, a heart or a lung.

Hence, as an embodiment of the model shape information obtaining step, a model 3D data obtaining step of obtaining model 3D data will be described. The model 3D data obtaining step includes a model image capturing step of capturing an image of a model with a medical image capturing device to obtain model image data, and a model 3D data generating step of generating model 3D data based on the model image data and the shape of the model. The model 3D data obtaining step may include a model property measuring step of measuring the model by MRE to obtain a model property.

### (i) Model image capturing step

The model image capturing step is a step of capturing an image of a model with a medical image capturing device to obtain model image data. Through the model image capturing step, the medical image capturing device obtains model shape information. The medical image capturing device is a device same as those that can be used in the medical image capturing step described above. Examples of the medical image capturing device include a CT device, a MRI device, and ultrasonic diagnostic equipment.

The medical image capturing device performs slice tomography of capturing cross-sectional images of a model a plurality of times. In this way, the medical image capturing device can obtain a plurality of model image data, which represent model images, which are images representing cross-sections of a model, as in the medical image capturing step described above. It is preferable that each of the plurality of model image data (also referred to as "model image data set") be an image of a DICOM-format, which is an international standard relating to medical image exchange. The model image data include image density information indicating image densities obtained by the medical image capturing device. When a CT device is used as the medical image capturing device, the image density is a CT value (X ray transmittance). When an MRI device is used as the medical image capturing device, the image density is an MRI signal value. When ultrasonic diagnostic equipment is used as the medical image capturing device, the image density is a reflection intensity.

As the medical image capturing device, any of a CT device, an MRI device, and ultrasonic diagnostic equipment may be used for capturing an image of an external shape of a model. It is suitable to use an MRI device among the medical image capturing devices, because a signal source of an MRI device is a hydrogen nucleus, and when the image capturing target is a model containing a hydrogel, the MRI device can capture an image of an internal shape of the model in addition to an image of the external shape of the model. "Containing a hydrogel" means that at least a part of the model is formed of a hydrogel. It is suitable to use an MRI device also because the MRI device can perform MRE measurement in the model property measuring step described below.

### (ii) Model property measuring step

The model property measuring step is a step of obtaining a model property of a model such as viscoelasticity or distribution of viscoelasticity by, for example, MRE measurement of the model. MRE measurement is a measurement by the same method as the MRE measurement in the biological property measuring step descried above. That is, it is preferable that a model property obtained by MRE measurement of a model be viscoelasticity. Examples of the method for obtaining the viscoelasticity of a model include not only the MRE measurement method, but also an ultrasonic elastography measurement method. In this regard, information obtained by ultrasonic elastography measurement is one-dimensional information, whereas information obtained by MRE measurement is two-dimensional information. Therefore, MRE measurement is preferable because it is easier to generate 3D data. MRE measurement is often included in the system of an MRI device as an optional function. Hence, MRE measurement is preferable also because the model image capturing step and the model property measuring step can be performed by the same MRI device. Incidentally, the model image capturing step by an MRI device and the model property measuring step by an MRI device can be performed almost at the same time. It is preferable to perform these steps almost at the same time, because this makes it possible to obtain accurate information of a model since model image data and a model property are obtained almost at the same time. "Almost at the same time" includes, for example, a case where the period of time for the model image capturing step and the period of time for the model property measuring step at least partially overlap, and a case where both of the model image capturing step and the model property measuring step can be performed by use of an MRI device once.

The model property measuring step is a step of obtaining a model property by MRE measurement of a model as described above. This means that as one main component among the materials constituting the model, the model contains water suitable for MRE measurement in which a hydrogen nucleus is a signal source. That is, it is preferable that the model contain a hydrogel described below.

The medical image capturing device includes a bed on which the image capturing target person lies. When capturing an image of or measuring a model of, for example, an excised organ in the model image capturing step or the model property measuring step, the model may be put directly on the bed. However, it is preferred to put the model indirectly. Indirectly putting the model means providing a support table between the model and the bed or putting the model on the bed in a state that the model is being dipped in a liquid or a gel. This makes it possible to capture an image of or measure the model while preventing deformation of the model due to contact with the bed. The support table needs at least to be able to prevent deformation of the model due to contact with the bed. Examples of the support table include a soft gel-state table.

### (iii) Model 3D data generating step

The model 3D data generating step is a step of generating model 3D data based on model image data obtained in the model image capturing step and a model property obtained in the model property measuring step. In the present disclosure, "model property information" is information indicating the model property obtained by MRE measurement of a model.

The model 3D data is information that includes a plurality of voxels generated based on the model image data, voxel-by-voxel image density information indicating an image density in the model image data and allocated to each voxel, and voxel-by-voxel model property information indicating a model property allocated to each voxel. In other words, the model 3D data is information that associates voxels, image density information, and model property information with one another. Note that the model property measuring step is an optional step of the model 3D data generating step. When the model property measuring step is not provided, model 3D data may be generated based only on a plurality of voxels generated based on the model image data, and image density information indicating an image density in the model image data and allocated to each voxel. In this case, the model 3D data is information including a plurality of voxels generated based on the model image data and image density information indicating an image density in the model image data and allocated to each voxel.

The method for generating the model 3D data will be specifically described.

First, the medical image capturing device or an image processing device relating to the medical image capturing device (both also referred to as "medical image capturing device, etc.") generates model 3D data for 3D image processing, from the model image data obtained in the model image capturing step. Like the medical 3D data described above, the model 3D data is an aggregate of voxels including at least one minimum unit such as a cube, and various kinds of information can be associated with each voxel.

Next, the medical image capturing device, etc. associates image density information indicating an image density in the model image data obtained in the model image capturing step with each voxel. Then, the medical image capturing device, etc. associates model property information indicating the model property obtained in the model property measuring step with each voxel. In this way, model 3D data, which is information associating voxels, image density information, and model property information with one another, can be generated, like the medical 3D data described above. Using the image density information, the model 3D data can be regionally divided (segmented) in the voxel units. This enables, for example, recognition, structural division, and 3D image analyses of shapes of the model including an external shape of the model and an internal shape of the model based on the model 3D data, and, for example, makes it possible to obtain model 3D data of a predetermined portion from the model 3D data of the model.

In the present disclosure, like the region-specific medical 3D data described above, model 3D data of a partial region obtained by voxel region division of the model 3D data is referred to as region-specific model 3D data. Region-specific model 3D data is one concept encompassed in the model 3D data. "Model shape information" is information representing a shape of a model obtained by measuring the model by, for example, MRI. Examples of the model shape information include information associating voxels and image density information with each other in the model 3D data, and information associating voxels and a model shape with each other.

### (3) Evaluation step

The evaluation method of the present disclosure includes an evaluation step of evaluating accuracy of a model with respect to an organism based on organism shape information representing the shape of the organism and model shape information representing the shape of the model. The accuracy of a model with respect to an organism indicates how correctly the model can reproduce the organism. Particularly, the evaluation step is a step of evaluating how correctly the shape of the model can reproduce the shape of the organism, and can evaluate the accuracy by, for example, calculating and displaying a concrete difference between organism shape information and model shape information using a computer and comparing a threshold with the calculated difference to judge which is the greater than the other. The evaluation step may also judge presence or absence of any difference between the organism shape information and the model shape information. The evaluation step may evaluate the accuracy based on the full data of the organism shape information and the model shape information, or may evaluate the accuracy based on partial data of the organism shape information and the model shape information or on a voxel-by-voxel basis. Moreover, images of predetermined cross-sections may be generated based on the organism shape information and the model shape information, and the images may be displayed by superimposition of the images or side-by-side arrangement of the images, or the images may be printed so that a judge can superimpose the images or arrange the images side by side and observe the images.

The evaluation method of the present disclosure evaluates accuracy based at least on shapes, but may also evaluate accuracy based on physical properties such as viscoelasticity and other properties in addition to shapes.

The evaluation step is preferably a step of evaluating accuracy of a model with respect to an organism based on organism shape information included in medical 3D data and model shape information included in model 3D data. As described above, it is preferable to obtain organism shape information as information included in medical 3D data and obtain model shape information as information included in model 3D data. This step may be performed by a human or by an information processing device such as a personal computer.

The method for evaluating accuracy of a model with respect to an organism based on organism shape information and model shape information will be specifically described.

As the method for evaluating accuracy of a model with respect to an organism, for example, MATERIALISE MIMICS available from Materialise NV can be used. In this case, organism shape information included in medical 3D data and model shape information included in model 3D data are overlaid with each other, and accuracy is evaluated based on difference between the shape information. In another method, accuracy is evaluated by judging whether an organism shape and a model shape are substantially the same as each other at corresponding parts of the organism and the model.

Examples of the corresponding parts of an organism and a model include: a part of the organism and a part of the model located at almost the same coordinates when coordinate systems are allocated to the organism and the model on the same basis; and a part having a specific structure in an organism (e.g., a tumor) and a part in a model imitating the part having the specific structure in the organism (e.g., a part imitating the tumor).

Examples of the criterion for judging whether an organism shape and a model shape are substantially the same as each other at corresponding parts of the organism and the model include a difference of within 5% between the model shape and the organism shape at the corresponding parts. The difference is not limited to within 5% but may be appropriately selected depending on the degree of accuracy needed, and may be, for example, within 50%, within 40%, within 30%, within 20%, and within 10% but is preferably as small as possible because it is possible to evaluate that the model is highly accurate.

When evaluating accuracy of a model with respect to an organism, it is preferable to compare not only external shapes at corresponding parts of the organism and the model but also internal shapes. This is for suppressing influences that may be given on the evaluation result by deformation of external shapes occurring when the organism and the model are put on the bed of the medical image capturing device. Examples of the method for comparing internal shapes and evaluating accuracy include a method of designating a portion of an external shape and a portion of an internal shape and comparing relative relationship of the positions, and a method of designating a portion of an internal shape and a portion of an internal shape and comparing relative relationship of the positions.

Examples of a portion of an external shape include a root that is present on the surface of an organ and from which a large-bore blood vessel connecting to another organ extends, and a surface of a bone. Examples of a portion of an internal shape include a position at some centimeters from a branch of a blood vessel.

In order to compare organism shape information and model shape information at corresponding parts of the organism and the model as described above, it is preferable to have also information indicating the position in the organism at which the organism shape is measured, and information indicating the position in the model at which the model shape is measured. Hence, when evaluating the accuracy of the model with respect to the organism, it is preferable to use medical 3D data, which is information associating voxels and image density information with each other, and model 3D data, which is information associating voxels and image density information with each other, and compare organism shape information and model shape information included in these data respectively. This is because voxels correspond to the information indicating the position.

### 2. Model producing method

The method for producing a model to be evaluated by the evaluation method of the present disclosure will be described.

The producing method is not particularly limited, and examples of the producing method include a method of producing a model using a 3D printer, and a method of producing a model by injecting a material into a template. The method of producing a model using a 3D printer is preferable. It is more preferable that the producing method include a producing step of producing a three-dimensional object using a 3D printer based on medical 3D data of an organism. When the producing method includes such a producing step, the producing method may include as needed, a medical 3D data obtaining step of obtaining medical 3D data of an organism, and a generating step of generating 3D data for object production based on the medical 3D data, as the steps performed before the producing step. As an example of the model producing method, a case where the method includes a medical 3D data obtaining step, a generating step, and a producing step will be described below.

In the present disclosure, "production of a three-dimensional object using a 3D printer based on medical 3D data of an organism" is not limited to production of a three-dimensional object by direct use of the medical 3D data, such as by inputting the medical 3D data into the 3D printer, but also includes production of a three-dimensional object by indirect use of the medical 3D data, such as by inputting 3D data for object production, which is generated based on the medical 3D data, into the 3D printer.

### (1) Medical 3D data obtaining step

The producing method preferably includes a medical 3D data obtaining step of obtaining medical 3D data of an organism. The medical 3D data obtaining step of the model producing method includes a medical image capturing step of capturing an image of an organism with a medical image capturing device to obtain medical image data, a biological property measuring step of measuring the organism by MRE to obtain a biological property, and a medical 3D data generating step of a generating medical 3D data based on medical image data and the biological property. The same descriptions as made on each step of the medical 3D data obtaining step described as an embodiment of the organism shape information obtaining step can be applied as descriptions on each of these steps.

### (2) Generating step

The producing method preferably includes a generating step of generating 3D data for object production based on the medical 3D data. In the present disclosure, "3D data for object production" means data generated based on the medical 3D data and input into a 3D printer. 3D data for object production may be formed of one data or a plurality of data. Two specific examples of the method for generating 3D data for object production will be described below. However, the method for generating 3D data for object production is not limited to these examples.

### (i) Generation of 3D data for object production including STL-format data

Generation of 3D data for object production including Standard Triangulated Language (STL)-format data based on the medical 3D data will be described.

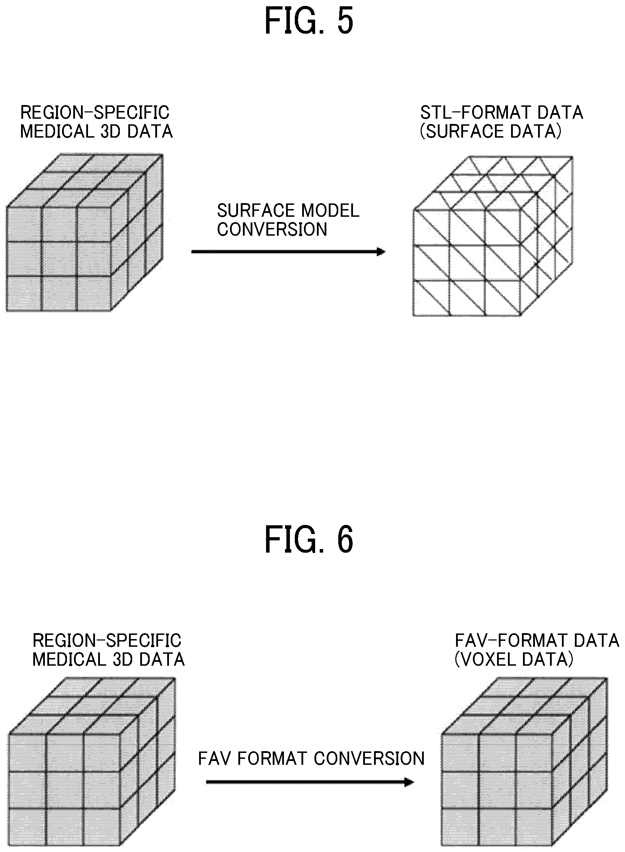

In this case, as illustrated in FIG. 5, region-specific medical 3D data obtained by voxel region division of the medical 3D data is converted to a surface model, to generate STL-format data corresponding to the region-specific medical 3D data. Because the STL format-data is surface data, the biological property information allocated to each voxel of the region-specific medical 3D data is lost through the surface model conversion. Hence, separately from the surface model conversion, biological property information for 3D object production including position information indicating the position of a voxel and biological property information allocated to each position information is generated based on the region-specific medical 3D data. Biological property information allocated to each position information means biological property information that has been associated with a voxel that has been present at the position indicated by the position information. That is, 3D data for object production as used herein includes STL-format data and biological property information for 3D object production.

### (ii) Generation of 3D data for object production including FAV-format data

Generation of 3D data for object production including FAbricatable Voxel (FAV)-format data based on the medical 3D data will be described.

In this case, as illustrated in FIG. 6, region-specific medical 3D data obtained by voxel region division of the medical 3D data is converted to a FAV format, to generate FAV-format data corresponding to the region-specific medical 3D data. Unlike STL-format data, FAV-format data is not surface data, but is defined as voxel data. Therefore, the medical 3D data, which is voxel data to which, for example, image density information and biological property information are allocated, can be converted to FAV-format data with, for example, the image density information and the biological property information kept allocated. That is, unlike the conversion to STL-format data, advantageously, it is possible to skip the step of separately performing data processing relating to necessary information such as biological property information.

### (3) Producing step

The producing method preferably includes a producing step of producing a three-dimensional object using a 3D printer based on the medical 3D data of an organism. As described above, in the present disclosure, "production of a three-dimensional object using a 3D printer based on the medical 3D data of an organism" is not limited to production of a three-dimensional object by direct use of the medical 3D data, such as by inputting the medical 3D data into the 3D printer, but also includes production of a three-dimensional object by indirect use of the medical 3D data, such as by inputting 3D data for object production, which is generated based on the medical 3D data, into the 3D printer.

The producing step will be described below, regarding production of a three-dimensional object by inputting 3D data for object production generated based on the medical 3D data into a 3D printer, as an example.

First, a case where the above-described 3D data for object production including STL-format data is input into the 3D printer will be described. In this case, the 3D data for object production includes STL-format data and biological property information for 3D object production. These data may be input simultaneously or separately.

The 3D printer converts the input STL-format data to a 2D image data set for printing. The 2D image data set for printing is a data set including a plurality of 2D image data for printing. The 2D image data for printing are two-dimensional slice data obtained by slicing the STL-format data at intervals defined by the resolution of the 3D printer in the Z axis direction.

Next, the 3D printer allocates strength property information indicating a strength property to each region of the 2D image data for printing based on the input biological property information for 3D object production. The 3D printer repeats a step of discharging a plurality of kinds of object producing compositions to predetermined regions in predetermined discharging amounts based on the 2D image data for printing and the strength property information for each region in the 2D image data for printing, and curing the object producing compositions. As a result, a three-dimensional object having a distribution of the strength property corresponding to the distribution of the biological property can be produced. Here, in the present disclosure, the "strength property" means a property of a three-dimensional object produced by a 3D printer, the property being corresponding to the biological property. For example, when the biological property is viscoelasticity, the strength property is also viscoelasticity. So long as 3D data for object production includes STL-format data, a three-dimensional object having a shape of an organism corresponding to the organism shape can be produced.

Next, a case where the above-described 3D data for object production including FAV-format data is input into the 3D printer will be described.

The 3D printer converts the input FAV-format data to a 2D image data set for printing, which is the same slice data as described above except that strength property information indicating a strength property of each region is allocated to each 2D image data for printing included in the 2D image data set for printing, based on the biological property information included in the FAV-format data. The 3D printer repeats a step of discharging a plurality of kinds of object producing compositions to predetermined regions in predetermined discharging amounts based on the 2D image data for printing and the strength property information for each region in the 2D image data for printing, and curing the object producing compositions. As a result, a three-dimensional object having a distribution of the strength property corresponding to the distribution of the biological property can be produced.

Examples of the 3D printer used in the producing step include a printer that can control the strength property of a three-dimensional object to be produced, region by region of the three-dimensional object, by discharging a plurality of kinds of object producing compositions to predetermined regions in predetermined discharging amounts. Specific examples of such a 3D printer include a 3D printer of a material jetting type (MJ type) configured to discharge the object producing compositions from inkjet heads.

Examples of the method for bringing correspondence between the strength property and the biological property as described above using a 3D printer include a method of using a 3D printer that can express gradation of the strength property. Hence, an example of this method will be described.

First, a high-strength object producing composition that can produce a three-dimensional object having a high strength property and a low-strength object producing composition that can produce a three-dimensional object having a low strength property are prepared. For each of these object producing compositions, at least one kind of a composition or a plurality of kinds of compositions may be used. It is preferable that the strength property of a three-dimensional object to be produced with the high-strength object producing composition be greater than or equal to the maximum biological property value in the organism. It is preferable that the strength property of a three-dimensional object to be produced with the low-strength object producing composition be less than or equal to the minimum biological property value in the organism.

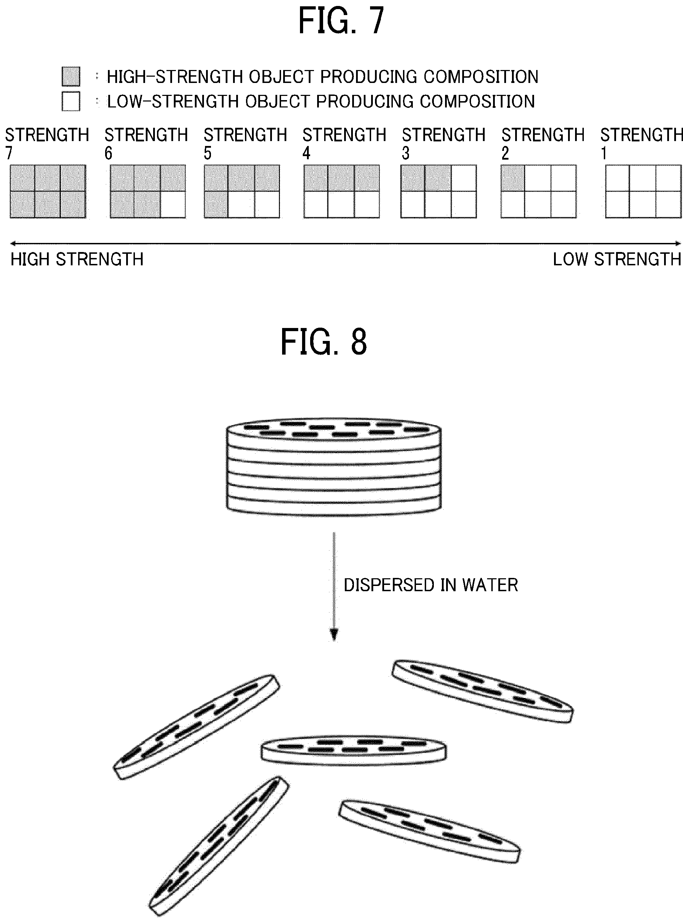

Next, the strength property of the three-dimensional object is controlled based on the pattern according to which the object producing compositions are arranged in order for a certain grid region to be filled with the object producing compositions. For example, as illustrated in FIG. 7, when expression of the strength property by seven gradations is needed, seven-gradation strength can be expressed by combination patterns according to which a grid, whose minimum unit is formed of six segments, is filled with the high-strength object producing composition and the low-strength object producing composition. By increasing the number of segments in a unit region of the grid, it is possible to realize various gradation expressions. A strength distribution in a horizontal direction can be set by arrangement of the compositions in the XY plane, and a strength distribution in the vertical direction can be set by arrangement of the compositions in the Z direction. This makes it possible to produce a three-dimensional object having a distribution of the strength property corresponding to the distribution of the biological property. That is, in the present disclosure, "a distribution of the strength property corresponding to the distribution of the biological property" is not limited to a distribution in which a strength property is equal to a biological property at the corresponding position, but also includes a distribution in which a strength property realized by gradation expression is approximate to a biological property at the corresponding position. "Approximate" means, for example, a difference of within 5% between the strength property (the same as a model property described below) and the biological property.

This method is efficient with voxel formats such as FAV, because voxel formats enable voxel expression. The number of liquid droplets of the object producing compositions for filling the unit segment is optional.

By previously knowing the strength properties of the gradations that can be expressed by a 3D printer by, for example, MRE measurement, it is possible to more accurately produce a three-dimensional object having a distribution of the strength property corresponding to the distribution of the biological property.

### 3. Object producing composition

The object producing composition used in the producing step will be described. The object producing composition is a composition prepared as a precursor for producing an object with a 3D printer. The object producing composition is preferably a composition that can produce a three-dimensional object having a distribution of a strength property corresponding to the distribution of the biological property. Examples of the object producing composition include a composition prepared as a precursor for producing an object to be formed of, for example, elastomer and gel. Above all, a composition prepared as a precursor for producing an object to be formed of hydrogel that contains, as one main component among constituent materials, water like an organism is preferable (such a composition will be referred to as "three-dimensional hydrogel object producing composition"). A three-dimensional hydrogel object producing composition as an example of the object producing composition will be described below.

The three-dimensional hydrogel object producing composition contains water and a polymerizable compound, and as needed, may contain a mineral, an organic solvent, a metal ion, and other components.

In the present disclosure, a "three-dimensional hydrogel object producing composition" is a liquid composition that cures and forms a hydrogel in response to irradiation with active energy rays such as light or heat, and that is used particularly for producing a three-dimensional object containing a hydrogel. In the present disclosure, a "hydrogel" means a structural body formed by water being embraced in a three-dimensional network structure containing a polymer. When such a three-dimensional network structure is a three-dimensional network structure formed by a polymer being combined with a mineral, the three-dimensional network structure is referred to particularly as "organic-inorganic-combined hydrogel". A hydrogel contains water as a main component. Specifically, a hydrogel contains water preferably by 30.0% by mass or greater, more preferably by 40.0% by mass or greater, and yet more preferably by 50.0% by mass or greater relative to the total amount of the hydrogel.

### (1) Water

The three-dimensional hydrogel object producing composition contains water. The water is not particularly limited, and any water that is ordinarily used as solvents may be used. Examples of the water include pure water such as ion-exchanged water, ultrafiltrated water, reverse osmotic water, and distilled water, and ultrapure water.

The content of the water may be appropriately selected depending on the intended purpose. For example, when the three-dimensional hydrogel object producing composition is used for producing a medical model, the content of the water is preferably 30.0% by mass or greater but 90.0% by mass or less and more preferably 40.0% by mass or greater but 90.0% by mass or less relative to the total amount of the three-dimensional hydrogel object producing composition.

Any other component such as an organic solvent may be dissolved or dispersed in the water in order to, for example, impart a moisture retaining property, an antibacterial activity, and conductivity, and adjust hardness.

### (2) Polymerizable compound

The three-dimensional hydrogel object producing composition contains a polymerizable compound, which is a compound containing a polymerizable functional group. Examples of the polymerizable compound include monomers and oligomers. The polymerizable compound polymerizes and forms at least part of a polymer in response to irradiation with active energy rays or heat. That is, a polymer contains a structural unit attributable to the polymerizable compound. A polymer crosslinks and combines with a mineral to form a three-dimensional network structure in a hydrogel. It is preferable that the polymerizable compound be water-soluble. For example, water solubility means a solubility of a monomer by 90% by mass or greater thereof when the monomer (1 g) is mixed and stirred in water (100 g) at 30 degrees C.

The polymerizable compound is not particularly limited so long as the polymerizable compound is a compound containing a polymerizable functional group. A compound containing a photopolymerizable functional group is preferable. In the present disclosure, a "polymerizable functional group" means a functional group that undergoes a polymerization reaction in response to irradiation with active energy rays or application of heat, and a "photopolymerizable functional group" particularly means a functional group that undergoes a polymerization reaction in response to irradiation with active energy rays. The photopolymerizable functional group is not limited to as described above, and examples of the photopolymerizable functional group include, but are not limited to, groups containing an ethylenic unsaturated bond, such as a (meth)acryloyl group, a vinyl group, and an allyl group; and cyclic ether groups such as an epoxy group. Specific examples of the compound that contains a group containing an ethylenic unsaturated bond include, but are not limited to, compounds containing a (meth)acrylamide group, (meth)acrylate compounds, compounds containing a (meth)acryloyl group, compounds containing a vinyl group, and compounds containing an allyl group.

### (i) Monomer

A monomer that can be used as the polymerizable compound is a compound that contains one or more polymerizable functional groups, a preferable example of which is an unsaturated carbon-carbon bond. Examples of the monomer include, but are not limited to, monofunctional monomers and multifunctional monomers. Examples of the multifunctional monomers include, but are not limited to, bifunctional monomers and trifunctional or higher monomers. One of these monomers may be used alone or two or more of these monomers may be used in combination.

### (A) Monofunctional monomer

Examples of the monofunctional monomer include, but are not limited to, acrylamide, N-substituted acrylamide derivatives, N,N-disubstituted acrylamide derivatives, N-substituted methacrylamide derivatives, N,N-disubstituted methacrylamide derivatives, acrylic acid, 2-ethylhexyl (meth)acrylate, 2-hydroxyethyl (meth)acrylate, 2-hydroxypropyl (meth)acrylate, caprolactone-modified tetrahydrofurfuryl (meth)acrylate, 3-methoxybutyl (meth)acrylate, tetrahydrofurfuryl (meth)acrylate, lauryl (meth)acrylate, 2-phenoxyethyl (meth)acrylate, isodecyl (meth)acrylate, isooctyl (meth)acrylate, tridecyl (meth)acrylate, caprolactone (meth)acrylate, ethoxylated nonylphenol (meth)acrylate, potassium acrylate, zinc acrylate, potassium methacrylate, magnesium acrylate, calcium acrylate, zinc methacrylate, magnesium methacrylate, aluminum acrylate, neodymium methacrylate, sodium methacrylate, and potassium acrylate. One of these monofunctional monomers may be used alone or two or more of these monofunctional monomers may be used in combination. Among these monofunctional monomers, acrylamide, N,N-dimethyl acrylamide, N-isopropyl acrylamide, acryloylmorpholine, hydroxyethyl acrylamide, and isobornyl (meth)acrylate are preferable.

The content of the monofunctional monomer is preferably 0.5% by mass or greater but 30.0% by mass or less relative to the total amount of the three-dimensional hydrogel object producing composition.

### (B) Bifunctional monomer

Examples of the bifunctional monomer include, but are not limited to, tripropylene glycol di(meth)acrylate, triethylene glycol di(meth)acrylate, tetraethylene glycol di(meth)acrylate, polypropylene glycol di(meth)acrylate, neopentyl glycol hydroxypivalic acid ester di(meth)acrylate, hydroxypivalic acid neopentyl glycol ester di(meth)acrylate, 1,3-butanediol di(meth)acrylate, 1,4-butanediol di(meth)acrylate, 1,6-hexanediol di(meth)acrylate, 1,9-nonanediol di(meth)acrylate, diethylene glycol di(meth)acrylate, neopentyl glycol di(meth)acrylate, tripropylene glycol di(meth)acrylate, caprolactone-modified hydroxypivalic acid neopentyl glycol ester di(meth)acrylate, propoxylated neopentyl glycol di(meth)acrylate, ethoxy-modified bisphenol A di(meth)acrylate, polyethylene glycol 200 di(meth)acrylate, and polyethylene glycol 400 di(meth)acrylate. One of these bifunctional monomers may be used alone or two or more of these bifunctional monomers may be used in combination.

### (C) Trifunctional or higher monomer

Examples of the trifunctional or higher monomer include, but are not limited to, trimethylolpropane tri(meth)acrylate, pentaerythritol tri(meth)acrylate, dipentaerythritol hexa(meth)acrylate, triallyl isocyanurate, ε-caprolactone-modified dipentaerythritol tri(meth)acrylate, ε-caprolactone-modified dipentaerythritol tetra(meth)acrylate, ε-caprolactone-modified dipentaerythritol penta(meth)acrylate, ε-caprolactone-modified dipentaerythritol hexa(meth)acrylate, tris(2-hydroxyethyl)isocyanurate tri(meth)acrylate, ethoxylated trimethylolpropane tri(meth)acrylate, propoxylated trimethylolpropane tri(meth)acrylate, propoxylated glyceryl tri(meth)acrylate, pentaerythritol tetra(meth)acrylate, ditrimethylolpropane tetra(meth)acrylate, dipentaerythritol hydroxypenta (meth)acrylate, ethoxylated pentaerythritol tetra(meth)acrylate, and penta(meth)acrylate ester. One of these trifunctional or higher monomers may be used alone or two or more of these trifunctional or higher monomers may be used in combination.

The content of the multifunctional monomer is preferably 0.01% by mass or greater but 10.0% by mass or less relative to the total amount of the three-dimensional hydrogel object producing composition.

### (ii) Oligomer

An oligomer is a low polymer of the monofunctional monomer described above or a low polymer that contains a reactive unsaturated bond group at an end thereof. One of such oligomers may be used alone or two or more of such oligomers may be used in combination.

### (3) Mineral

It is preferable that the three-dimensional hydrogel object producing composition contain a mineral. The mineral is not particularly limited and may be appropriately selected depending on the intended purpose so long as the mineral can bond with a polymer formed of the polymerizable compound described above. Examples of the mineral include layered clay minerals, and water-swellable layered clay minerals in particular.

A water-swellable layered clay mineral will be described with reference to FIG. 8. FIG. 8 is an exemplary view illustrating an example of a water-swellable layered clay mineral serving as the mineral, and a dispersed state of the water-swellable layered clay mineral in water. As illustrated in the upper section of FIG. 8, a water-swellable layered clay mineral is present in a state of single layers, and has a multilayered state of two-dimensional disk-shaped crystals having a unit lattice in the crystals. When the water-swellable layered clay mineral in the upper section of FIG. 8 is dispersed in water, the respective single layers separate into a plurality of two-dimensional disk-shaped crystals as illustrated in the lower section of FIG. 8.

Water swellability means a property of a layered clay mineral being dispersed in water with water molecules inserted between the respective single layers thereof as illustrated in FIG. 8. The shape of the single layers of the water-swellable layered clay mineral is not limited to the disk shape. The single layers of the water-swellable layered clay mineral may have any other shape.

Examples of the water-swellable layered clay mineral include water-swellable smectite, and water-swellable mica. More specific examples of the water-swellable layered clay mineral include water-swellable hectorite containing sodium as an interlayer ion, water-swellable montmorillonite, water-swellable saponite, and water-swellable synthetic mica. One of these water-swellable layered clay minerals may be used alone or two or more of these water-swellable layered clay minerals may be used in combination. Among these water-swellable layered clay minerals, water-swellable hectorite is preferable because a highly elastic hydrogel can be obtained.

The water-swellable hectorite may be an appropriately synthesized product or a commercially available product. Examples of the commercially available product include synthetic hectorite (LAPONITE XLG, available from Rock Wood), SWN (available from Coop Chemical Ltd.), and fluorinated hectorite SWF (available from Coop Chemical Ltd.). Among these commercially available products, synthetic hectorite is preferable in terms of improving the elastic modulus of a hydrogel.

The content of the mineral is preferably 1.0% by mass or greater but 40.0% by mass or less relative to the total amount of the three-dimensional hydrogel object producing composition.

### (4) Organic solvent

The three-dimensional hydrogel object producing composition may contain an organic solvent as needed. The organic solvent is contained in order to, for example, improve the moisture retaining property of a hydrogel.

Examples of the organic solvent include alkyl alcohols containing from one through four carbon atoms, amides, ketones, ketone alcohols, ethers, polyvalent alcohols, polyalkylene glycols, lower alcohol ethers of polyvalent alcohols, alkanolamines, and N-methyl-2-pyrrolidone. One of these organic solvents may be used alone or two or more of these organic solvents may be used in combination.

Among these organic solvents, polyvalent alcohols are preferable in terms of a moisture retaining property. Specifically, polyvalent alcohols such as ethylene glycol, propylene glycol, 1,2-propanediol, 1,2-butanediol, 1,3-butanediol, 1,4-butanediol, diethylene glycol, triethylene glycol, 1,2,6-hexanetriol, thioglycol, hexylene glycol, and glycerin can be suitably used.

The content of the organic solvent is preferably 1.0% by mass or greater but 10.0% by mass or less relative to the total amount of the three-dimensional hydrogel object producing composition.

### (5) Metal ion

The three-dimensional hydrogel object producing composition may contain a metal ion as needed. A metal ion is an ionized metal element, and is typically added in the form of a metal salt. A metal salt is a generic term for compounds obtained by substituting a metal ion for a hydrogen atom of an acid.

Examples of the metal salt include, but are not limited to, monovalent metal salts, divalent metal salts, and trivalent metal salts. Among these metal salts, divalent or higher multivalent metal salts are preferable because divalent or higher multivalent metal salts can form a stronger cross-linked structure and produce a model having a high breaking stress and a high elongation degree.

Examples of the monovalent metal salt include, but are not limited to, a lithium salt, a sodium salt, and a potassium salt.

Examples of the divalent metal salt include, but are not limited to, a calcium salt, a magnesium salt, a nickel salt, a divalent iron salt, a copper salt, a manganese salt, a cobalt salt, a zinc salt, a cadmium salt, and a beryllium salt.

Examples of the trivalent metal salt include, but are not limited to, an aluminum salt, a trivalent iron salt, a gallium salt, a neodymium salt, a gadolinium salt, and a cerium salt.

It is preferable that the metal salt be ionic. Examples of a metal ion that constitutes a metal salt include, but are not limited to, alkali metal ions such as Li⁺, Na⁺, and K⁺; alkali earth metal ions such as Be²⁺, Mg²⁺, and Ca²⁺; transition metal ions such as Cu²⁺, Fe³⁺, Ni²⁺, Mn²⁺, and Co²⁺; base metal ions such as Al³⁺, Ga³⁺, Zn²⁺, and Cd ²⁺; and lanthanoid ions such as Nd³⁺, Gd³⁺, and Ce³⁺.

Examples of the divalent iron salt include, but are not limited to, iron (II) chloride. Examples of the calcium salts include, but are not limited to, calcium nitrate, calcium chloride, and calcium acetate. Examples of the magnesium salt include, but are not limited to, magnesium chloride, magnesium acetate, magnesium sulfate, and magnesium nitrate. Examples of the nickel salt include, but are not limited to, nickel chloride. Examples of the aluminum salt include, but are not limited to, aluminum nitrate. These salts may be anhydrides or hydrates.

### (6) Other components

The three-dimensional hydrogel object producing composition may contain other components as needed.

The other components are not particularly limited and may be appropriately selected depending on the intended purpose. Examples of the other components include a stabilizer, a surface treating agent, a polymerization initiator, a colorant, a viscosity modifier, a tackifier, an antioxidant, an age resister, a cross-linking agent, an ultraviolet absorbent, a plasticizer, a preservative, metal ions, a filler, metal particles, and a dispersant.

### (i) Stabilizer

A stabilizer is contained in order to disperse the mineral stably and maintain the three-dimensional hydrogel object producing composition in a sol state. When the three-dimensional hydrogel object producing composition is used in a system configured to discharge the three-dimensional hydrogel object producing composition in the form of a liquid droplet, a stabilizer is contained in order to stabilize the properties of the three-dimensional hydrogel object producing composition as a liquid.

Examples of the stabilizer include high-concentration phosphates, glycols, and nonionic surfactants.

### (ii) Surface treating agent

Examples of the surface treating agent include polyester resins, polyvinyl acetate resins, silicone resins, coumarone resins, fatty acid esters, glycerides, and waxes.

### (III) Polymerization initiator

Examples of the polymerization initiator include thermal polymerization initiators and photopolymerization initiators. Of these polymerization initiators, photopolymerization initiators that produce radicals or cations in response to irradiation with active energy rays are preferable in terms of storage stability.

As a photopolymerization initiator, an arbitrary substance that produces radicals in response to irradiation with light (particularly, ultraviolet rays having a wavelength of 220 nm or longer but 400 nm or shorter) can be used.

The thermal polymerization initiator is not particularly limited and may be appropriately selected depending on the intended purpose. Examples of the thermal polymerization initiator include azo-based initiators, peroxide initiators, persulfate initiators, and redox (oxido-reduction) initiators.

### (7) Properties of three-dimensional hydrogel object producing composition

The viscosity of the three-dimensional hydrogel object producing composition at 25 degrees C is preferably 3.0 mPa·s or higher but 20.0 mPa·s or lower and more preferably 6.0 mPa·s or higher but 12.0 mPa·s or lower. When the viscosity of the three-dimensional hydrogel object producing composition is 3.0 mPa·s or higher but 20.0 mPa·s or lower, the three-dimensional hydrogel object producing composition can be suitably applied to liquid droplet discharging by 3D printers (particularly, of a material jetting type). The viscosity can be measured with, for example, a rotary viscometer (VISCOMATE VM-150III, available from Toki Sangyo Co., Ltd.).

The surface tension of the three-dimensional hydrogel object producing composition is preferably 20 mN/m or higher but 45 mN/m or lower and more preferably 25 mN/m or higher but 34 mN/m or lower. When the surface tension of the three-dimensional hydrogel object producing composition is 20 mN/m or higher, discharging stability of the three-dimensional hydrogel object producing composition can be improved. When the surface tension of the three-dimensional hydrogel object producing composition is 45 mN/m or lower, the three-dimensional hydrogel object producing composition can be easily filled into, for example, discharging nozzles for object production. The surface tension can be measured with, for example, a surface tensiometer (AUTOMATIC CONTACT ANGLE METER DM-701, available from Kyowa Interface Science Co., Ltd.).

### 4. Method for producing three-dimensional object using 3D printer

As an example of the method for producing a three-dimensional object with a 3D printer (hereinafter, may also be referred to as "object producing apparatus"), a method for producing a three-dimensional hydrogel object using the three-dimensional hydrogel object producing composition described above in a 3D printer of a material jetting type will be described.

The method for producing a three-dimensional hydrogel object by a material jetting method includes a liquid film forming step of applying a liquid droplet of the three-dimensional hydrogel object producing composition to form a liquid film, and a curing step of curing the liquid film of the three-dimensional hydrogel object producing composition, and repeats the liquid film forming step and the curing step sequentially. The three-dimensional hydrogel object producing method may include, as needed, a support producing step of producing a support for supporting a three-dimensional hydrogel object and other steps.

A three-dimensional hydrogel object producing apparatus of a material jetting type includes a storing unit storing the three-dimensional hydrogel object producing composition, a liquid film forming unit configured to apply a liquid droplet of the three-dimensional hydrogel object producing composition stored, to form a liquid film, and a curing unit configured to cure the liquid film of the three-dimensional hydrogel object producing composition, and repeats the formation of a liquid film by the liquid film forming unit and curing by the curing unit sequentially.

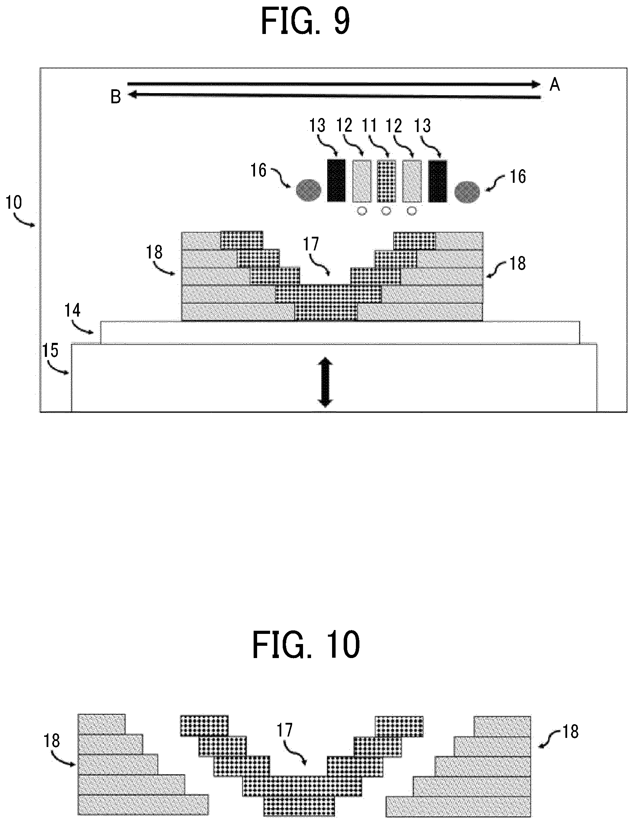

The material jetting method will be described with reference to FIG. 9 and FIG. 10. FIG. 9 is an exemplary view illustrating an example of the three-dimensional hydrogel object producing apparatus. FIG. 10 is an exemplary view illustrating an example of a three-dimensional hydrogel object detached from supports. The three-dimensional hydrogel object producing apparatus 10 of a material jetting type illustrated in FIG. 9 uses head units in which inkjet heads are arranged, and is configured to cause a three-dimensional hydrogel object producing composition discharging head unit 11 to discharge a three-dimensional hydrogel object producing composition stored in a three-dimensional hydrogel object producing composition storing container and cause support producing composition discharging head units 12 to discharge a support producing composition stored in a support producing composition storing container to an object support substrate 14, and cause adjacent ultraviolet irradiators 13 to cure the three-dimensional hydrogel object producing composition and the support producing composition, to laminate layers. The support producing composition is a liquid composition that cures in response to irradiation with active energy rays such as light or heat, to produce a support for supporting a three-dimensional hydrogel object. Examples of the support producing composition include acrylic-based materials. The object producing apparatus 10 may include a smoothing member 16 configured to smooth the three-dimensional hydrogel object producing composition discharged.

The object producing apparatus 10 is configured to laminate layers while lowering a stage 15 in accordance with the number of layers laminated, in order to keep the three-dimensional hydrogel object producing composition discharging head unit 11, the support producing composition discharging head units 12, and the ultraviolet irradiators 13 at a constant gap from the three-dimensional object (hydrogel) 17 and the supports (support materials) 18.

The ultraviolet irradiators 13 of the object producing apparatus 10 are used in the travels in the directions of both of the arrows A and B. Heat generated along with ultraviolet irradiation smooths the surfaces of the laminated layers, and the dimensional stability of the three-dimensional object 17 is improved as a result.

After object production by the object producing apparatus 10 is completed, the three-dimensional object 17 and the supports 18 are drawn in the horizontal direction as illustrated in FIG. 10. As a result, the supports 18 are detached as integrated bodies, and the three-dimensional object 17 can be taken out easily.

### (1) Liquid film forming step

The method for applying the three-dimensional hydrogel object producing composition in the liquid film forming step is not particularly limited and may be appropriately selected depending on the intended purpose so long as the method can apply a liquid droplet to an intended position with a suitable accuracy. A known liquid droplet discharging method can be used. Specific examples of the liquid droplet discharging method include a dispenser method, a spray method, and an inkjet method. The inkjet method is preferable.

The volume of a liquid droplet of the three-dimensional hydrogel object producing composition is preferably 2 pL or greater but 60 pL or less and more preferably 15 pL or greater but 30 pL or less. When the volume of a liquid droplet is 2 pL or greater, discharging stability can be improved. When the volume of a liquid droplet is 60 pL or less, for example, discharging nozzles for object production can be easily filled with the three-dimensional hydrogel object producing composition.

### (2) Curing step

Examples of the curing unit configured to cure the liquid film of the three-dimensional hydrogel object producing composition in the curing step include ultraviolet (UV) irradiation lamps, and electron beams. Examples of the kinds of the ultraviolet (UV) irradiation lamps include high-pressure mercury lamps, ultrahigh-pressure mercury lamps, and metal halides.

As the curing unit for curing the three-dimensional hydrogel object producing composition, an Ultra Violet-Light Emitting Diode (UV-LED) can be suitably used. The emission wavelength of the LED is not particularly limited and is typically, for example, 365 nm, 375 nm, 385 nm, 395 nm, and 405 nm. Considering influences of colors on the object, shorter wavelength emission is more advantageous for increasing absorption by an initiator. Moreover, the UV-LED generates a lower thermal energy during curing and can save thermal damage on the hydrogel, compared with ultraviolet irradiation lamps commonly used (e.g., high-pressure mercury lamps, ultrahigh-pressure mercury lamps, and metal halides) and electron beams. This effect is particularly remarkable for three-dimensional hydrogel objects to be produced with the three-dimensional hydrogel object producing composition, because such hydrogels are used in a state of containing water.

The three-dimensional hydrogel object producing method includes a liquid film forming step of applying a liquid droplet of the three-dimensional hydrogel object producing composition to form a liquid film and a curing step of curing the liquid film of the three-dimensional hydrogel object producing composition, and repeats the liquid film forming step and the curing step sequentially. The number of times of repetition is not particularly limited and may be appropriately selected depending on, for example, the size and shape of the three-dimensional hydrogel object to be produced. The average thickness per cured layer is preferably 10 micrometers or greater but 50 micrometers or less. When the average thickness is 10 micrometers or greater but 50 micrometers or less, it is possible to produce an object accurately while suppressing peel or detachment.

### (3) Support producing step

The support producing composition used in the support producing step is a liquid composition that cures in response to irradiation with active energy rays such as light or heat, to produce a support for supporting a three-dimensional hydrogel object. The support producing composition is compositionally different from the three-dimensional hydrogel object producing composition. Specifically, it is preferable that the support producing composition contain, for example, a curable material and a polymerization initiator, and be free of water and a mineral. The curable material is a compound that undergoes a polymerization reaction and cures in response to, for example, irradiation with active energy rays (e.g., ultraviolet rays and electron beams) and heating, and examples of the curable material include active-energy-ray-curable compounds and thermosetting compounds. Among these curable materials, materials that are liquid at normal temperature are preferable.

The support producing composition is applied to a position different from the position to which the three-dimensional hydrogel object producing composition is applied. This means that the support producing composition and the three-dimensional hydrogel object producing composition do not overlap with each other. The support producing composition and the three-dimensional hydrogel object producing composition may adjoin each other. Examples of the method for applying the support producing composition include the same as the methods for applying the three-dimensional hydrogel object producing composition.

### (4) Other steps

Examples of the other steps include a step of smoothing a liquid film, a detaching step, a polishing step of polishing a three-dimensional object, and a washing step of washing a three-dimensional object. It is preferable to include the step of smoothing a liquid film. This is because the liquid film formed in the liquid film forming step does not always have the intended film thickness (layer thickness) at all positions. For example, when forming a liquid film by an inkjet method, there may occur, for example, discharging failure or inter-dot height difference, making it difficult to form a highly accurate three-dimensional object. To such a problem, conceivable methods include a method of mechanically smoothing (leveling) a liquid film after formed, a method of mechanically scraping away a hydrogel thin film obtained through curing of the liquid film, and a method of sensing the degree of smoothness and adjusting the amount of film formation on a dot level during lamination of the next layer.

When producing a three-dimensional hydrogel object as an organ model, the method of mechanically leveling a liquid film is preferable as the smoothing method because the hydrogel serving as the material constituting the organ model is relatively soft. Examples of the mechanical smoothing method include a leveling method using a blade-shaped member and a leveling method using a roller-shaped member.

### (5) Method for producing three-dimensional hydrogel object including parts having different strength properties

Next, as a more specific description of the three-dimensional hydrogel object producing method, an example of a method for producing a three-dimensional hydrogel object including parts having different strength properties will be described. The following description will be made taking as an example, an embodiment where two kinds of compositionally different three-dimensional hydrogel object producing compositions are used. However, this embodiment is non-limiting. A person ordinarily skilled in the art would easily understand another embodiment (for example, an embodiment where three or more kinds of three-dimensional hydrogel object producing compositions are used) based on the following description.

The method for producing a three-dimensional hydrogel object including parts having different strength properties includes a liquid film forming step of separately applying liquid droplets of a plurality of compositionally different three-dimensional hydrogel object producing compositions to form a liquid film including a plurality of compositionally different regions, and a curing step of curing the liquid film, and repeats the liquid film forming step and the curing step sequentially.

The object producing method described above employs a three-dimensional hydrogel object producing apparatus that includes storing units separately storing a plurality of compositionally different three-dimensional hydrogel object producing compositions, a liquid film forming unit configured to separately apply liquid droplets of the plurality of compositionally different three-dimensional hydrogel object producing compositions stored, to form a liquid film including a plurality of compositionally different regions, and a curing unit configured to cure the liquid film, where the three-dimensional object producing apparatus repeats the formation of a liquid film by the liquid film forming unit and the curing by the curing unit sequentially.

Specifically, using a first three-dimensional hydrogel object producing composition and a second three-dimensional hydrogel object producing composition compositionally different from the first three-dimensional hydrogel object producing composition, a liquid film continuously including a plurality of compositionally different regions is formed based on control on the positions to which and the amounts by which liquid droplets of the respective three-dimensional hydrogel object producing compositions are applied. The first three-dimensional hydrogel object producing composition is an example of the high-strength object producing composition described above, and the second three-dimensional hydrogel object producing composition is an example of the low-strength object producing composition. Next, the liquid film is cured, to form a cured film for one layer continuously including the regions described above. Subsequently, the formation of a liquid film and the curing are repeated sequentially, to laminate cured films and produce a three-dimensional hydrogel object continuously including a plurality of parts having different strength properties. The plurality of parts having different strength properties in the three-dimensional hydrogel object may be present by having different strength properties in a cured film for one layer or may be present by having different strength properties between cured films.

### 5. Use of model

Examples of the use of the model include an organ model imitating an organ. A human organ model is preferable. An organ means a functional organ constituting an organism. Therefore, the organ is not limited to internal organs, but also encompasses all kinds of organs that constitute an organism, such as bones, skin, and blood vessels.

The use of the organ model is roughly classified into two types. One is an organ model in a general-purpose use, and the other is an organ model in an individual-specific use. The organ model in a general-purpose use is an organ model used for, for example, performance validation, calibration, and training in medical device development, and structure confirmation in the educational settings. In this case, the organ model has an average shape and average properties of a target organ. Also in this case, it is preferable that the organ model be a model that is produced based mainly on data of a healthy person and based on data of a single person or average data of a plurality of persons. The organ model in an individual-specific use is an organ model used for, for example, informed consent, preoperative simulations, and operative method training in the medical settings. In this case, the organ model has the shape and properties of the organ that includes the affected part of the target individual patient. Also in this case, it is preferable that the organ model be a model that is produced based on data of the individual patient and reproduces the affected part.

When a model is used as a human organ model, the content of water among materials constituting the model, such as a hydrogel is preferably 70% by mass or greater but 85% by mass or less relative to the total amount of the organ model. When the content of water is 70% by mass or greater but 85% by mass or less, the water content in the human organ model can be equal or similar to the water content in the target actual human organ, and the human organ model can become suitable for use. Specifically, the content of water in a human heart model is preferably about 80% by mass, the content of water in a human kidney model is preferably about 83% by mass, and the content of water in a human brain or bowel model is preferably about 75% by mass. Accordingly, the content of water in a model is more preferably 75% by mass or greater but 83% by mass or less relative to the total amount of the model.

### [Examples]

The present disclosure will be described below by way of Examples. The present disclosure should not be construed as being limited to these Examples.

### <Production of a high-strength object producing composition A>

First, ion-exchanged water was subjected to pressure reducing deaeration for 30 minutes to prepare pure water. To the pure water (580.0 parts by mass) under stirring, synthetic hectorite (LAPONITE RD, obtained from Byk Additives & Instruments, Inc.) serving as a water-swellable clay mineral (67.0 parts by mass) was added little by little, and the resultant was further stirred to produce a mixture liquid. Next, to the mixture liquid, etidronic acid (obtained from Tokyo Chemical Industry Co., Ltd.) (5.0 parts by mass) was added as a dispersant of the synthetic hectorite, to obtain a dispersion liquid.

Next, to the obtained dispersion liquid, dimethyl acrylamide (DMAA, obtained from Tokyo Chemical Industry Co., Ltd.) (262.0 parts by mass) having passed through an active alumina column to remove any polymerization initiator was added as a monomer. To the resultant, N,N'-methylene bisacrylamide (MBAA, obtained from Tokyo Chemical Industry Co., Ltd.) (2.4 parts by mass) and polyethylene glycol diacrylate (A-400, obtained from Shin-Nakamura Chemical Co., Ltd.) (8.0 parts by mass) were added as cross-linking agents. To the resultant, glycerin (obtained from Sakamoto Yakuhin Kogyo Co., Ltd.) (300.0 parts by mass) was added as an anti-drying agent, and the resultant was mixed.

Next, to the resultant, N,N,N',N'-tetramethyl ethylene diamine (TEMED, obtained from Tokyo Chemical Industry Co., Ltd.) (6.7 parts by mass) was added as a polymerization accelerator. To the resultant, EMULGEN LS-106 (obtained from Kao Corporation) (5.3 parts by mass) was added as a surfactant, and the resultant was mixed.

Next, to the resultant under cooling in an ice bath, a solution (12.3 parts by mass) of a photopolymerization initiator (IRGACURE 184, obtained from BASF GmbH) in 4% by mass of methanol was added. The resultant was stirred and mixed and subsequently subjected to pressure reducing deaeration for 20 minutes. Successively, the resultant was filtrated to remove, for example, impurities, to obtain a homogeneous high-strength object producing composition A.

### -Measurement of viscoelasticity of cured product of high-strength object producing composition A-

First, a hydrogel was produced using the high-strength object producing composition A. Specifically, a container having a size of 31 mm×31 mm (with a thickness of 10 mm) was prepared and filled with the high-strength object producing composition A. Then, the high-strength object producing composition A was cured using an ultraviolet irradiator (obtained from Ushio Inc., SPOT CURE SP5-250DB). The irradiation conditions include a wavelength of 365 nm, an irradiation intensity of 350 mJ/cm² , and an irradiation time of 60 seconds.

Next, the physical properties of the produced hydrogel were measured with a rheometer. As a result, the storage modulus was 8,320 Pa and the loss modulus was 2,540 Pa.

### <Production of low-strength object producing composition B>

First, ion-exchanged water was subjected to pressure reducing deaeration for 30 minutes to prepare pure water. To the pure water (580.0 parts by mass) under stirring, synthetic hectorite (LAPONITE RD, obtained from Byk Additives & Instruments, Inc.) serving as a water-swellable clay mineral (67.0 parts by mass) was added little by little, and the resultant was further stirred to produce a mixture liquid. Next, to the mixture liquid, etidronic acid (obtained from Tokyo Chemical Industry Co., Ltd.) (5.0 parts by mass) was added as a dispersant of the synthetic hectorite, to obtain a dispersion liquid.

Next, to the obtained dispersion liquid, dimethyl acrylamide (DMAA, obtained from Tokyo Chemical Industry Co., Ltd.) (262.0 parts by mass) having passed through an active alumina column to remove any polymerization initiator was added as a monomer. To the resultant, N,N'-methylene bisacrylamide (MBAA, obtained from Tokyo Chemical Industry Co., Ltd.) (0.6 parts by mass) and polyethylene glycol diacrylate (A-400, obtained from Shin-Nakamura Chemical Co., Ltd.) (2.0 parts by mass) were added as cross-linking agents. To the resultant, glycerin (obtained from Sakamoto Yakuhin Kogyo Co., Ltd.) (300.0 parts by mass) was added as an anti-drying agent, and the resultant was mixed.

Next, to the resultant, N,N,N',N'-tetramethyl ethylene diamine (TEMED, obtained from Tokyo Chemical Industry Co., Ltd.) (6.7 parts by mass) was added as a polymerization accelerator. To the resultant, EMULGEN LS-106 (obtained from Kao Corporation) (5.3 parts by mass) was added as a surfactant, and the resultant was mixed.

Next, to the resultant under cooling in an ice bath, a solution (12.3 parts by mass) of a photopolymerization initiator (IRGACURE 184, obtained from BASF GmbH) in 4% by mass of methanol was added. The resultant was stirred and mixed and subsequently subjected to pressure reducing deaeration for 20 minutes. Successively, the resultant was filtrated to remove, for example, impurities, to obtain a homogeneous low-strength object producing composition B.

### -Measurement of viscoelasticity of cured product of low-strength object producing composition B-

First, a hydrogel was produced using the low-strength object producing composition B. Specifically, a container having a size of 31 mm×31 mm (with a thickness of 10 mm) was prepared and filled with the low-strength object producing composition B. Then, the low-strength object producing composition B was cured using an ultraviolet irradiator (obtained from Ushio Inc., SPOT CURE SP5-250DB). The irradiation conditions include a wavelength of 365 nm, an irradiation intensity of 350 mJ/cm² , and an irradiation time of 60 seconds.

Next, the physical properties of the produced hydrogel were measured with a rheometer. As a result, the storage modulus was 4,415 Pa and the loss modulus was 3,104 Pa.

### <Obtainment of medical 3D data>

Using an MRI device capable of performing MRE measurement, medical 3D data including a liver part of a patient suffering from fatty liver was obtained. This medical 3D data was generated based on medical image data obtained by capturing an image of the patient with the MRI device and a biological property obtained by MRE measurement of the patient. Specifically, this medical 3D data includes a plurality of voxels generated based on the medical image data, image density information indicating an image density (MRI signal value) in the medical image data and allocated to each voxel, and biological property information indicating a biological property (viscoelasticity) allocated to each voxel.

The biological property (viscoelasticity) of the liver part obtained by MRE measurement was within the ranges of the viscoelasticity of a cured product of the high-strength object producing composition A and the viscoelasticity of a cured product of the low-strength object producing composition B at any local parts of the liver part.

### <Generation of 3D data for object production>

The medical 3D data was subjected to voxel region division, to obtain region-specific medical 3D data representing the liver part. Next, the region-specific medical 3D data was converted to a FAV format, to generate FAV-format data, which was 3D data for object production.

### <Object production example of liver model 1>

The high-strength object producing composition A and the low-strength object producing composition B were loaded into a 3D printer of a material jetting type as illustrated in FIG. 9 capable of expressing strength property gradations, and filled into inkjet heads of the 3D printer. Next, the 3D data for object production was input into the 3D printer, and a liver model 1, which was a three-dimensional object having a distribution of a strength property corresponding to the distribution of the biological property was produced based on the 3D data for object production. Specifically, the respective object producing compositions were discharged from the inkjet heads, and formation of a liquid film and curing were repeated sequentially, to produce the model.

### <Production of object producing composition C>

An object producing composition C was obtained in the same manner as in Production of high-strength object producing composition A, except that a solution of an initiator (potassium persulfate) in 4% by mass of water was used instead of the solution of a photopolymerization initiator (IRGACURE 184, obtained from BASF GmbH) in 4% by mass of methanol.

### <Object production example of liver model 2>

A casting mold imitating the liver part of a patient was produced with a 3D printer (FORM 2, obtained from Formlabs Inc.) based on the 3D data for object production. Next, the object producing composition C was injected into the casting mold and cured by being maintained at room temperature for 24 hours, to obtain a liver model 2.

### <Obtainment of model 3D data>

Using an MRI device capable of performing MRE measurement, model 3D data of the liver model 1 and the liver model 2 were obtained respectively. These model 3D data were generated based on model image data obtained by capturing images of the liver model 1 and the liver model 2 with the MRI device respectively and model properties obtained by MRE measurement of the liver model 1 and the liver model 2 respectively. Specifically, these model 3D data each include a plurality of voxels generated based on the model image data, image density information indicating an image density (MRI signal value) in the model image data and allocated to each voxel, and model property information indicating a model property (viscoelasticity) allocated to each voxel.

### [Evaluation of model property accuracy]

### (Example 1)

Using MATERIALISE MIMICS obtained from Materialise NV, accuracy of the liver model 1 with respect to the liver part was evaluated based on the biological property information included in the obtained medical 3D data of the liver part and the model property information included in the model 3D data of the liver model 1. Specifically, the difference between the model property and the biological property at corresponding parts of the liver part and the liver model 1 was calculated. As a result, the difference was 1%.

### (Example 2)

Using MATERIALISE MIMICS obtained from Materialise NV, accuracy of the liver model 2 with respect to the liver part was evaluated based on the biological property information included in the obtained medical 3D data of the liver part and the model property information included in the model 3D data of the liver model 2. Specifically, the difference between the model property and the biological property at corresponding parts of the liver part and the liver model 2 was calculated. As a result, the difference was 70%.

### [Evaluation of model shape accuracy]

### (Example 1)

Using MATERIALISE MIMICS obtained from Materialise NV, shape accuracy of the liver model 1 with respect to the liver part was evaluated based on the organism shape information included in the obtained medical 3D data of the liver part and the model shape information included in the model 3D data of the liver model 1. Specifically, the difference between the model shape and the organism shape at corresponding parts of the liver part and the liver model 1 was calculated. As a result, the difference was about 9%.

### (Example 2)

Using MATERIALISE MIMICS obtained from Materialise NV, shape accuracy of the liver model 2 with respect to the liver part was evaluated based on the organism shape information included in the obtained medical 3D data of the liver part and the model shape information included in the model 3D data of the liver model 2. Specifically, the difference between the model shape and the organism shape at corresponding parts of the liver part and the liver model 2 was calculated. As a result, the difference was about 4%.

In the way described above, the accuracy of the liver model 1 with respect to the liver part was evaluated as being high, and the accuracy of the liver model 2 with respect to the liver part was evaluated as being low.

The above-described embodiments are illustrative and do not limit the present invention. Thus, numerous additional modifications and variations are possible in light of the above teachings. For example, elements and/or features of different illustrative embodiments may be combined with each other and/or substituted for each other within the scope of the present invention as defined by the appended claims.

## Claims

1. An evaluation method comprising:
evaluating accuracy of a model containing a hydrogel with respect to an organism based on organism shape information representing an organism shape obtained by capturing an image of the organism by magnetic resonance imaging, model shape information representing a model shape obtained by capturing an image of the model by magnetic resonance imaging, biological property information indicating a biological property obtained by magnetic resonance elastography measurement of the organism, and model property information indicating a model property obtained by magnetic resonance elastography measurement of the model.

2. The evaluation method according to claim 1,
wherein the organism shape information includes organism internal shape information.

3. The evaluation method according to claim 1 or 2,
wherein the evaluating comprises evaluating the accuracy by comparison between the organism shape information included in medical 3D data and the model shape information included in model 3D data,
the medical 3D data is generated based on medical image data obtained by capturing the image of the organism with a medical image capturing device, and
the model 3D data is generated based on model image data obtained by capturing the image of the model with a medical image capturing device.

4. The evaluation method according to claim 3,
wherein the medical 3D data includes a plurality of voxels generated based on the medical image data, and image density information indicating an image density in the medical image data and allocated to each of the voxels, and
the model 3D data includes a plurality of voxels generated based on the model image data, and image density information indicating an image density in the model image data and allocated to each of the voxels.

5. The evaluation method according to any one of claims 2 to 4,
wherein the model is produced based on the organism internal shape information, and has an organism internal shape that corresponds to the organism internal shape information.

6. The evaluation method according to any one of claims 1 to 5,
wherein the model is a human organ model.

7. The evaluation method according to claim 1,
wherein the model is produced based on the biological property information, and has a distribution of a strength property corresponding to a distribution of the biological property.

8. The evaluation method according to any one of claims 1 to 7,
wherein the model is produced using a 3D printer of a material jetting type.

## Patentansprüche

1. Auswertungsverfahren, das Folgendes umfasst:
Auswerten einer Genauigkeit eines Modells, das ein Hydrogel enthält, in Bezug auf einen Organismus, basierend auf Informationen zur Organismusform, die eine Organismusform darstellen, die durch Aufnehmen eines Bilds des Organismus durch Magnetresonanztomographie erhalten wurde, Informationen zur Modellform, die eine Modellform darstellen, die durch Aufnehmen eines Bilds des Modells durch Magnetresonanztomographie erhalten wurde, Informationen zu biologischen Eigenschaften, die eine biologische Eigenschaft angeben, die durch eine Magnetresonanz-Elastographie-Messung des Organismus erhalten wurde, und Informationen zu Modelleigenschaften, die eine Modelleigenschaft angeben, die durch eine Magnetresonanz-Elastographie-Messung des Modells erhalten wurde.

2. Auswertungsverfahren nach Anspruch 1,
wobei die Informationen zur Organismusform Informationen zur inneren Form des Organismus beinhalten.

3. Auswertungsverfahren nach Anspruch 1 oder 2,
wobei das Auswerten ein Auswerten der Genauigkeit durch Vergleich zwischen den in medizinischen 3D-Daten beinhalteten Informationen zur Organismusform und den in 3D-Modelldaten beinhalteten Informationen zur Modellform umfasst,
die medizinischen 3D-Daten basierend auf medizinischen Bilddaten erzeugt werden, die durch Aufnehmen des Bilds des Organismus mit einer medizinischen Bildaufnahmevorrichtung erhalten werden, und
die 3D-Modelldaten basierend auf Modellbilddaten erzeugt werden, die durch Aufnehmen des Bilds des Modells mit einer medizinischen Bildaufnahmevorrichtung erhalten werden.

4. Auswertungsverfahren nach Anspruch 3,
wobei die medizinischen 3D-Daten eine Vielzahl von Voxeln beinhalten, die basierend auf den medizinischen Bilddaten erzeugt wurden, und Bilddichteinformationen, die eine Bilddichte in den medizinischen Bilddaten angeben und jedem der Voxel zugeordnet sind, beinhalten, und
wobei die 3D-Modelldaten eine Vielzahl von Voxeln beinhalten, die basierend auf den Modellbilddaten erzeugt wurden, und Bilddichteinformationen, die eine Bilddichte in den Modellbilddaten angeben und jedem der Voxel zugeordnet sind, beinhalten.

5. Auswertungsverfahren nach einem der Ansprüche 2 bis 4,
wobei das Modell basierend auf den Informationen zur inneren Form des Organismus erstellt wird und eine innere Form des Organismus aufweist, die den Informationen zur inneren Form des Organismus entspricht.

6. Auswertungsverfahren nach einem der Ansprüche 1 bis 5,
wobei das Modell ein Modell eines menschlichen Organs ist.

7. Auswertungsverfahren nach Anspruch 1,
wobei das Modell basierend auf den Informationen zu biologischen Eigenschaften erstellt wird und eine Verteilung einer Stärkeeigenschaft aufweist, die einer Verteilung der biologischen Eigenschaft entspricht.

8. Auswertungsverfahren nach einem der Ansprüche 1 bis 7,
wobei das Modell unter Verwendung eines Material-Jetting-3D-Druckers hergestellt wird.

## Revendications

1. Procédé d'évaluation comprenant :
l'évaluation de la précision d'un modèle contenant un hydrogel par rapport à un organisme sur la base d'informations de forme d'organisme représentant une forme d'organisme obtenue en capturant une image de l'organisme par imagerie par résonance magnétique, d'informations de forme de modèle représentant une forme de modèle obtenue en capturant une image du modèle par imagerie par résonance magnétique, d'informations de propriété biologique indiquant une propriété biologique obtenue par mesure d'élastographie par résonance magnétique de l'organisme, et d'informations de propriété de modèle indiquant une propriété de modèle obtenue par mesure d'élastographie par résonance magnétique du modèle.

2. Procédé d'évaluation selon la revendication 1,
dans lequel les informations de forme d'organisme incluent des informations de forme interne d'organisme.

3. Procédé d'évaluation selon la revendication 1 ou 2,
dans lequel l'évaluation comprend l'évaluation de la précision par comparaison entre les informations de forme d'organisme incluses dans des données médicales 3D et les informations de forme de modèle incluses dans des données de modèle 3D,
les données médicales 3D sont générées sur la base de données d'image médicale obtenues en capturant l'image de l'organisme avec un dispositif de capture d'images médicales, et
les données de modèle 3D sont générées sur la base de données d'image de modèle obtenues en capturant l'image du modèle avec un dispositif de capture d'images médicales.

4. Procédé d'évaluation selon la revendication 3,
dans lequel les données médicales 3D incluent une pluralité de voxels générés sur la base des données d'image médicale, et des informations de densité d'image indiquant une densité d'image dans les données d'image médicale et attribuées à chacun des voxels,
et
les données de modèle 3D incluent une pluralité de voxels générés sur la base des données d'image de modèle, et des informations de densité d'image indiquant une densité d'image dans les données d'image de modèle et attribuées à chacun des voxels.

5. Procédé d'évaluation selon l'une quelconque des revendications 2 à 4,
dans lequel le modèle est produit sur la base des informations de forme interne d'organisme, et présente une forme interne d'organisme qui correspond aux informations de forme interne d'organisme.

6. Procédé d'évaluation selon l'une quelconque des revendications 1 à 5,
dans lequel le modèle est un modèle d'organe humain.

7. Procédé d'évaluation selon la revendication 1,
dans lequel le modèle est produit sur la base des informations de propriété biologique et présente une distribution d'une propriété de résistance correspondant à une distribution de la propriété biologique.

8. Procédé d'évaluation selon l'une quelconque des revendications 1 à 7,
dans lequel le modèle est produit à l'aide d'une imprimante 3D de type à projection de matériau.
